# EUROPÄISCHE PATENTSCHRIFT

(11) **EP 2 013 258 B1**
(45) Veröffentlichungstag und Bekanntmachung des Hinweises auf die Patenterteilung: **25.08.2010**
(21) Anmeldenummer: 07727687.1
(22) Anmeldetag: 03.04.2007
(51) Int. Cl.: C08G 18/76, C07C 263/20, C08G 18/12

(54) **VERFAHREN ZUR HERSTELLUNG VON POLYURETHANEN**
PROCESS FOR PREPARING POLYURETHANES
PROCEDE DE FABRICATION DE POLYURETHANNES

(30) Priorität: 12.04.2006 EP 06112525
(43) Veröffentlichungstag der Anmeldung: 14.01.2009
(73) Patentinhaber: BASF SE, 67056 Ludwigshafen (DE)
(72) Erfinder: REESE, Hans-Jürgen, 49401 Damme (DE); MURRAR, Imbridt, 01968 Senftenberg (DE); FRITZ, Ralf, 49143 Bissendorf-schledehausen (DE); CABRERA, Andres, 49080 Osnabrück (DE); MAGG, Birgit, 49401 Damme (DE)
(86) Internationale Anmeldenummer: PCT/EP2007/053216
(87) Internationale Veröffentlichungsnummer: WO 2007/115971

(56) Entgegenhaltungen:
- EP-A1- 0 046 917
- EP-A1- 0 294 110
- EP-A1- 1 518 874
- DE-A1- 1 932 832
- JP-A- 10 158 231

## Beschreibung

Gegenstand der Erfindung ist ein Verfahren zur Herstellung von Polyurethanen durch Umsetzung von uretoniminarmen Polyisocyanaten mit Verbindungen mit mindestens zwei mit Isocyanatgruppen reaktiven Wasserstoffatomen.

Polyurethane sind seit langem bekannt und vielfach beschrieben. Sie können auf vielen technischen Gebieten eingesetzt werden. Ein wichtiges Anwendungsgebiet der Polyurethane sind Einkomponentenschäume aus Aerosolbehältern, auch als Aerosolschäume oder Montageschäume bezeichnet, sowie Kleb- und Dichtstoffe.

Bei diesen Anwendungsgebieten werden als Polyisocyanate zumeist Mischungen aus Diphenylmethandiisocyanat und Polyphenylenpolymethylenpolyisocyanaten, häufig auch als Roh-MDI oder Polymer-MDI bezeichnet, und/oder Umsetzungsprodukte aus Polyisocyanaten mit einem Unterschuss von Verbindungen mit mindestens zwei mit Isocyanatgruppen reaktiven Wasserstoffatomen, sogenannte NCO-Prepolymere, eingesetzt.

Bei den genannten Anwendungen wird häufig gefordert, den Gehalt an atembaren Isocyanaten in der Reaktionsmischung zu verringern.

Bei der Verwendung von Prepolymeren kann dies erreicht werden, indem nach der Umsetzung die nicht umgesetzten monomeren Isocyanate entfernt werden, beispielsweise durch Destillation.

Bei Polyurethanen für den Einsatz in Aerosolschäumen wird als Polyisocyanat zumeist Polymer-MDI eingesetzt. Dieses enthält üblicherweise einen hohen Anteil an 2-Kern-MDI. Die Aerosol- oder Montageschäume sind insbesondere Einkomponentenschäume.

Aerosolschäume sind im Bereich des Bauwesens häufig angewandte Montagemittel zum Einbau von Fenstern und Türen in Bauwerken sowie als Füllmaterial für bautechnisch bedingte Hohlräume oder Mauerdurchbrüche für Rohrinstallationen. Ein solcher Aerosolbehälter beinhaltet ein Prepolymer sowie Treibmittel und Zusätze. Durch Austragen seines Inhaltes mittels Treibmittel, seinem Aufschäumen durch Verdampfen des Treibmittels, die sogenannte Frothwirkung, und durch seine Aushärtung mit Luftfeuchtigkeit, entsteht der gewünschte Schaum.

Einkomponentenschäume auf der Basis von NCO-haltigen Prepolymeren sind die bekanntesten Schäume dieser Art. Es gibt hierbei unterschiedliche Produkte, die je nach Zusammensetzung zu harten bis weich-elastischen Schäumen führen.

Nachteilig bei all diesen Formulierungen ist, dass erhebliche Mengen an monomerem Isocyanat in diesen NCO-haltigen Prepolymeren vorhanden sind, wodurch während des Schäumprozesses ein gewisses Gefährdungspotential durch atembares Isocyanat bestehen kann. Bekannt sind in dieser Gruppe von Schäumen aber auch Formulierungen mit deutlich reduzierten Gehalten an freien monomeren Isocyanaten.

So ist nach EP 1 518 874 bekannt, dass ein monomerarmes Isocyanat zur Fertigung von Einkomponentenschäumen verwendet wird, welches aus einem definierten Polyphenylen-polymethylenpolyisocyanat durch destillative Entfernung des monomeren Isocyanats gewonnen wird. Durch Einsatz dieses Produktes gegebenenfalls im Gemisch mit Verdünnungsmitteln und weiteren isocyanatgruppenhaltigen Verbindungen werden so monomerarme Einkomponentenschäume erhalten. Nachteilig hierbei ist, dass so gefertigte Einkomporienten-Aerosolschäume wenig lagerstabil sind, wodurch der Inhalt der Aerosoldruckbehälter innerhalb weniger Wochen fest und damit unbrauchbar wird.

Auch WO 2005/007721 A1 beschreibt den Einsatz von Gemischen aus monomerarmen NCO-endständigen Prepolymeren, d.h. von Monomeren befreiten Umsetzungsprodukten aus Polyolen und Diphenylmethandiisocyanat im stöchiometrischen Überschuss, entmonomerisiertem Polyphenylenpolymethylenpolyisocyanat, trimerisiertem Hexamethylendiisocyanat und Verdünnungsmitteln. Nachteilig sind hier die zur Realisierung des geforderten verminderten Monomergehaltes an Isocyanat extrem hohen Viskositäten der Einsatzstoffe, die den Einsatz technologisch schwierig gestalten und die Tatsache, dass die Lagerstabilität wie bei der Lösung nach EP 1 518 874 nicht gewährleistet ist.

Aufgabe der Erfindung war es, monomerarme Isocyanatkomponenten auf Basis von Roh-MDI zu entwickeln, die eine verbesserte Lagerstabilität und gute Verarbeitungseigenschaften aufweisen.

Die Aufgabe konnte gelöst werden durch die Verwendung von Polyisocyanaten mit einer mittleren Funktionalität von größer 2, einem Gehalt an Diisocyanaten von maximal 2 Gew.-% und einem Gehalt an Uretoniminen von maximal 4 Gew.-%, jeweils bezogen auf das Gewicht des Polyisocyanats.

Gegenstand der Erfindung ist demzufolge ein Verfahren zur Herstellung von Polyurethanen durch Umsetzung von Polyisocyanaten a) mit Verbindungen mit mindestens zwei mit Isocyanatgruppen reaktiven Wasserstoffatomen b), **dadurch gekennzeichnet, dass** als Polyisocyanat a) mindestens ein Gemisch aus Diphenylmethandiisocyanat und Polyphenylenpolymethylenpolyisocyanaten mit einer mittleren Funktionalität von 2,2 bis 5,2, einem Gehalt an 2-Kern-MDI von maximal 2 Gew.-%, einem Gehalt an 3-Kern-MDI von 25 bis 65 Gew.-%, einem Gehalt an 4-Kern-MDI von 5 bis 45 Gew.-%, einem Gehalt an >5-Kern-MDI von 1 bis 40 Gew.-%, und einem Gehalt an Uretoniminen von maximal 4 Gew.-%, jeweils bezogen auf das Gewicht des Polyisocyanats ai), eingesetzt wird. Der Gehalt von Uretoniminen in Polymer-MDI wird mittels FT-IR-Analyse auf der Grundlage einer Kalibration mit 3-Kern Uretonimin ermittelt.

Gegenstand der Erfindung ist die Verwendung der Polyisocyanatmischung nach den Ansprüchen 9 und 10 zur Herstellung von Polyurethanen, insbesondere von 1-Komponenten-Polyurethan-Dosenschäumen, Polyurethan-Kleb- und/oder Dichtstoffen, Polyurethan-Elastomeren, 2-Komponenten-Polyurethanschäumen, insbesondere 2-Komponenten-Polyurethanhartschäumen.

Die erfindungsgemäßen Polyisocyanate ai) können, je nach Einsatzgebiet, allein oder im Gemisch mit anderen Polyisocyanaten zur Herstellung von Polyurethanen eingesetzt werden.

Die erfindungsgemäßen Polyisocyanate ai) können in Zweikomponentensystemen und Einkomponentensystemen eingesetzt werden. Bei Zweikomponentensystemen wird eine Verbindung mit mindestens zwei mit Isocyanatgruppen reaktiven Wasserstoffatomen mit einem Polyisocyanat zum Polyurethan umgesetzt.

Bei Einkomponentensystemen wird aus dem Polyisocyanat und einem Unterschuss der Verbindungen mit mindestens zwei mit Isocyanatgruppen reaktiven Wasserstoffatomen ein NCO-haltiges Prepolymer hergestellt, das beispielsweise, wie oben bei den Aerosolschäumen beschrieben, mit Luftfeuchtigkeit zum Polyurethan-Polyharnstoff aushärtet. Diese Ausführungsform wird insbesondere bei Aerosolschäumen angewandt. Bei Einkomponentensystemen wird das erfindungsgemäße Polyisocyanat ai) zumeist im Gemisch mit anderen Polyisocyanaten, vorzugsweise Isocyanatgruppen enthaltenden Prepolymeren verwendet.

Die Polyisocyanate ai) sind erhältlich durch Entfernung des Diphenylmethandiisocyanats und des Uretonimins aus einem Gemisch aus Diphenylmethandiisocyanat und Polyphenylenpolymethylen-polyisocyanaten, wobei vorteilhafterweise zuerst das Uretonimin und nachfolgend erst das Diphenylmethandiisocyanat zu entfernen sind. Die Entfernung des Diphenylmethandiisocyanats aus einem Gemisch ist beispielsweise beschrieben im EP 1 518 874. Nach dem Entmonomerisierungsverfahren analog der Lehre von EP 1 518 874 erhält man aus dem uretoniminarmen Polyphenylenpolymethylenpolyisocyanat-Gemisch ein Produkt mit einem L*-Wert von größer 90 und einem b*-Wert kleiner 70, bestimmt nach DIN 6162 und DIN 6164.

Wie beschrieben, wird das zur Herstellung des Polyisocyanats ai) eingesetzte Gemisch aus Diphenylmethandiisocyanat und Polyphenylenpolymethylenpolyisocyanaten zunächst von Nebenverbindungen, wie Uretonimin befreit. Diese werden bei der Herstellung und Aufarbeitung insbesondere durch thermische Belastung der Polyisocyanate gebildet. Diese Nebenverbindungen aus dem Herstellungsprozess, wie Uretdione, Uretonimine, Carbamoylchloride, sind in dem Ausgangspolyisocyanat zu maximal 25 Gew.-% enthalten. Die Entfernung erfolgt vorzugsweise durch Flüssig-Flüssig-Extraktion mit polaren oder unpolaren Lösungsmitteln. In einer besonderen Ausführungsform bevorzugt sind Kohlenwasserstoffe, wie Cyclohexan. Derartige Verfahren sind beispielsweise beschrieben in DE 15 43 258 oder EP 133 538.

In einer bevorzugten Ausführungsform der Abtrennung des Uretonimins wird das eingesetzte Polyphenylenpolymethylenpolyisocyanat, beispielsweise Lupranat^{®}M-Typen der BASF, mit Cyclohexan im Verhältnis von Isocyanat : Lösungsmittel von 1 : 1 bis 1 : 15, bevorzugt 1 : 1,5 bis 1 : 12 und besonders bevorzugt 1 : 2,5 bis 1 : 10 bei einer Temperatur von 20 bis 90 °C und bevorzugt 30 bis 80 °C für 1 bis 180 min und bevorzugt 5 bis 150 min in Kontakt gebracht. Danach wird das Produktgemisch bis zur vollständigen Phasenbildung bei 20 bis 40 °C und bevorzugt bei Raumtemperatur stehen gelassen. Die untere Phase ist das sogenannte "Raffinat", das das abzutrennende Uretonimin sowie höherkernige MDI-Homologe enthält. Die obere Phase ist das sogenannte "Extrakt", das das gewünschte uretoniminarme Polyphenylenpolymethylenpolyisocyanat und Lösungsmittel enthält. Beide Phasen werden getrennt und das Lösungsmittel mittels Vakuumdestillation vollständig entfernt. Der Restgehalt an Cyclohexan ist vorzugsweise kleiner 20 ppm.

Aus dem so behandelten Gemisch wird anschließend das monomere Diisocyanat abgetrennt. Die Entfernung des monomeren Diisocyanats kann vorzugsweise durch Destillation, vorzugsweise unter Vakuum, erfolgen. Dabei ist es bevorzugt, die Destillation mit einem Dünnschichtverdampfer oder einem Kurzwegverdampfer durchzuführen. Ein derartiges Verfahren ist beispielsweise in EP 1518874 beschrieben. Die Abtrennung der Monomere wird vorzugsweise bei einer Temperatur von unter 160°C, besonders bevorzugt 100 bis 158 °C und insbesondere 120 bis 155°C durchgeführt. Der Druck beträgt vorzugsweise 0,001 bis 10 mbar, besonders bevorzugt 0,01 bis 1 mbar und insbesondere 0,02 bis 0,9 mbar.

In einer bevorzugten Ausführungsform des erfindungsgemäßen Verfahrens hat das als Ausgangsprodukt eingesetzte Polymer-MDI-Gemisch folgende Zusammensetzung:

| | |
|---|---|
| 2-Kern MDI | 45,0 ± 25,0 % |
| 3-Kern MDI | 25,0 ± 10,0 % |
| 4-Kern MDI | 10,5 ± 5,5 % |
| ≥ 5-Kern MDI-Homologe | 17,5 ± 15,5 % |

Die aus diesem Ausgangsprodukt hergestellten erfindungsgemäßen Produkte haben einen Gehalt an Uretoniminen von maximal 4 Gew.-%, einen Gehalt an 2-Kern MDI von maximal 2 Gew.-%, einen Gehalt an 3-Kern MDI von 25 bis 65 Gew.-%, einen Gehalt an 4-Kern MDI von 5 bis 45 Gew.-% und einen Gehalt an ≥ 5-Kern MDI von 1 bis 40 Gew.-% und eine mittlere Funktionalität von 2,2 bis 5,2. Die Viskosität der erfindungsgemäßen uretonimin- und Monomer-MDI-armen Polyphenylenpolymethylenpolyisocyanate ist vorzugsweise kleiner 7000 mPa·s bei 25 °C und insbesondere 500 bis 5000 mPa·s bei 25 °C.

Aus diesem Produkt hergestellte Polyisocyanate ai) zeichnen sich neben ihrer guten Lagerstabilität unter anderem dadurch aus, dass sie eine vergleichsweise sehr geringe Eigenfärbung aufweisen und als höherfunktionelle Polyisocyanate vorteilhaft als Vernetzer, insbesondere in den 2-Komponenten-Polyurethan-Systemen, besonders bevorzugt in 2-Komponenten-PUR-Hartschaum-Systemen eingesetzt werden können.

Die hohe Lagerstabilität der nach der erfindungsgemäßen Lehre gefertigten Isocyanatkomponente ist sowohl für die 2-Komponenten-Anwendungen, als auch in der Verwendung als Isocyanatkomponente bei 1-Komponenten-Anwendungen, beispielsweise im Druckbehälter beim 1-Komponenten- Aerosolschaum gewährleistet.

Wie beschrieben, können die erfindungsgemäßen Polyisocyanate ai) allein oder in Kombination mit anderen Polyisocyanaten eingesetzt werden. Vorzugsweise erfolgt der Einsatz der Polyisocyanate ai) im Gemisch mit anderen Polyisocyanaten.

In einer bevorzugten Ausführungsform des erfindungsgemäßen Verfahrens wird gemeinsam mit dem Polyisocyanat ai) mindestens ein Urethangruppen und Isocynatgruppen enthaltendes Prepolymer aii) eingesetzt.

Derartige Verbindungen und ihre Herstellung sind bekannt. Sie werden üblicherweise durch Umsetzung von Polyisocyanaten mit einem stöchiometrischen Unterschuss an Verbindungen mit mindestens zwei mit Isocyanatgruppen reaktiven Wasserstoffatomen hergestellt. Als Polyisocyanate können die üblichen und bekannten Di- und Polyisocyanate eingesetzt werden. Beispielhaft seien genannt Toluylendiisocyanat (TDI), Diphenylmethandiisocyanat (MDI), Mischungen aus Diphenylmethandiisocyanat und Polyphenylenpolymethylenpolyisocyanaten (Roh-MDI), sowie aliphatische Diisocyanate, wie Hexamethylendiisocyanat (HDI) und Isophorondiisocyanat (IPDI). Besonders bevorzugt wird MDI oder Roh-MDI, insbesondere MDI eingesetzt. Als Verbindungen mit mindestens zwei mit Isocyanatgruppen reaktiven Wasserstoffatomen kommen zumeist mindestens difunktionelle Alkohole, vorzugsweise Polyetheralkohole, zum Einsatz. Besonders bevorzugt sind die Polyetheralkohole zwei- bis dreifunktionell und haben ein Molekulargewicht im Bereich zwischen 100 und 6000 g/mol.

Nach der Umsetzung werden die nicht umgesetzten Diisocyanate aus der Reaktionsmischung abgetrennt. Dies erfolgt üblicherweise durch Destillation, wobei die Destillation in einem Dünnschicht- oder einem Kurzwegverdampfer durchgeführt werden sollte. Derartige Verfahren sind beispielsweise bekannt aus DE 10 2004 038 784.

Da die Viskosität der Polyisocyanate ai) und aii) sowie der Mischungen aus diesen Verbindungen für viele Anwendungsgebiete zu hoch ist, ist es bevorzugt, den Polyisocyanaten ein inertes Verdünnungsmittel aiii) zuzusetzen. Als Komponente aiii) kommen solche Verbindungen in Frage, die gegenüber den zur Herstellung der Polyurethane eingesetzten Verbindungen inert sind. Geeignete Verbindungen sind beispielsweise hochsiedende Lösungsmittel, Weichmacher und Flammschutzmittel. Beispielsweise Diphenylkresylphosphat, Dioktylphthalat, Dioktyladipat, Triethylphosphat oder Trichloralkylphosphate. Flammschutzmittel werden insbesondere bei solchen Anwendungen als Weichmacher eingesetzt, bei deren Endprodukten ein Flammschutz erforderlich ist, beispielsweise beim Aerosolschaum.

Das Verhältnis der Komponenten ai), aii) und gegebenenfalls aiii) zueinander hängt von den Anforderungen an das Endprodukt ab.

Bei Aerosolschaum enthält die Isocyanatkomponente bevorzugt 20 bis 65 Gew.-% der Komponente ai), 15 bis 55 Gew.-% der Komponente aii) und 5 bis 65 Gew.-% der Komponente aiii). Bei der Verwendung als Vernetzer, insbesondere für die Herstellung von Polyurethan-Hartschaumstoffen, enthält die Isocyanatkomponente bevorzugt 20 bis 96 Gew.-% der Komponente ai), 0 bis 55 Gew.-% der Komponente aii) und 5 bis 65 Gew.-% der Komponente aiii). Beim Einsatz zur Herstellung von Kleb- und Dichtstoffen enthält die Isocyanatkomponente bevorzugt größer 0 bis 45 Gew.-% der Komponente ai), 35 bis 85 Gew.-% der Komponente aii) und 5 bis 65 Gew.-% der Komponente aiii).

Die Herstellung der Polyurethane unter Verwendung der erfindungsgemäßen Polyisocyanate erfolgt nach üblichen und bekannten Verfahren durch Umsetzung mit Verbindungen mit mindestens zwei mit Isocyanatgruppen reaktiven Wasserstoffatomen. Als Verbindungen mit mindestens zwei mit Isocyanatgruppen reaktiven Wasserstoffatomen können die hierfür üblichen und bekannten Verbindungen, insbesondere Polyetheralkohole, Polyesteralkohole, Polyetheresteralkohole und Polyetheramine verwendet werden.

Im Falle der 2-Komponenten-Verfahren werden die Mischungen aus ai) sowie gegebenenfalls aii) und aiii), gegebenenfalls im Gemisch mit weiteren Polyisocyanaten, mit Verbindungen mit mindestens zwei mit Isocyanatgruppen reaktiven Wasserstoffatomen, zum Polyurethan umgesetzt. Nach diesem Verfahren können kompakte Polyurethan-Reaktionsharze, bekannt als Polyurethangießharze, oder, unter Verwendung von Treibmitteln, Polyurethan-Hartschaumstoffe hergestellt werden. Als Verbindungen mit mindestens zwei mit Isocyanatgruppen reaktiven Wasserstoffatomen werden vorzugsweise Polyetheralkohole und/oder Polyesteralkohole mit einer Funktionalität von mindestens 3 und einer Hydroxylzahl im Bereich zwischen 100 und 700 mg KOH/g eingesetzt.

Bei der Herstellung von Hartschaumstoffen können zur Einstellung der Viskosität und Funktionalität der Polyisocyanatkomponente neben der Komponente ai) auch weitere nicht entmonomerisierte Gemische aus Zwei- und Mehrkern-MDI eingesetzt werden. Dies ist bei derartigen Anwendungen tolerierbar. Es können neben der Komponente ai) auch Prepolymere, vorzugsweise solche mit einer Funktionalität von größer 2, insbesondere 3 oder höher, eingesetzt werden.

Die 1-Komponenten-Verfahren kommen insbesondere bei der Herstellung von Aerosolschäumen zum Einsatz. Hierbei werden die Polyisocyanate mit einem Unterschuss an Verbindungen mit zwei mit Isocyanatgruppen reaktiven Wasserstoffatomen zu einem Prepolymeren umgesetzt und das so entstandene Prepolymer mit einem Treibmittel in einen Druckbehälter gefüllt. Dazu werden weitere Verbindungen mit mindestens zwei mit Isocyanatgruppen reaktiven Wasserstoffatomen dosiert, so dass im Druckbehälter ein Prepolymer mit einem geringeren Gehalt an freien Isocyanatgruppen entsteht.

Durch die Verwendung der erfindungsgemäßen Polyisocyanate ai) können lagerstabile Polyisocyanatmischungen mit einem geringen Gehalt an flüchtigen Diisocyanaten bereitgestellt werden, die auch in lagerstabilen 1-Komponenten-PUR-Aerosolschäumen eingesetzt werden können.

Die Polyisocyanate ai) sind heller und haben eine geringere Viskosität als solche Produkte, die keiner Extraktion unterzogen wurden. Ausgehend von einem Polymer-MDI (Lupranat^{®}M20) mit einer Farbzahl von 20 Jod, einem L*-Wert von 85,6 und einem b*-Wert von 70,1 wird nach der extraktiven Entfernung von Uretoniminen und anderen Nebenverbindungen ein Polymer-MDI mit einer Farbzahl von 0,8 Jod, einem L*-Wert von 99,3 und einem b*-Wert von 5,1 erhalten.

Die Erfindung soll an den nachstehenden Beispielen näher beschrieben werden.

### Ausführungsbeispiel 1:

### Fertigung eines ein NCO-funktionalisierten Umsetzungsproduktes aus Diphenylmethandiisocyanat und einer OH-funktionellen Verbindung (Einsatzstoff aii)

In einem Reaktor wurden 4,4'-MDI und ein Polypropylenglykolgemisch mit der mittleren Molmasse 900 unter inerten Bedingungen in einem Mol-Verhältnis von 4,6 zu 1 bei 65 °C zur Reaktion gebracht. Nach einer Stunde Nachreaktion bei 65 °C wurde ein Prepolymerprodukt mit einem NCO-Gehalt von 15,5 Gew.-%, einer Viskosität von 1963 mPa·s bei 25 °C und 312 mPa·s bei 50 °C und einem Monomer-MDI-Gehalt von 47,6 % erhalten. Dieses Prepolymer wurde in einem zweistufigen Destillationsprozess bei 0,17 mbar und 190 °C sowie 0,03 mbar und 168 °C entmonomerisiert. Das erhaltene monomerarme MDI-Prepolymer hatte einen NCO-Gehalt von 5,8 Gew.-%, eine Viskosität von 5973 mPa·s bei 50 °C und einen Monomer-MDI-Gehalt von kleiner 0,1 %.

### Ausführungsbeispiel 2:

### Fertigung eines entmonomerisierten uretoniminarmen Polyphenylenpolymethylenpolyisocyanats (Einsatzstoff ai)

Ein Polyphenylenpolymethylenpolyisocyanat (BASF: Lupranat^{®} M20) mit einem Monomer-MDI-Gehalt von 37 %, einem NCO-Gehalt von 31,2 Gew.-%, einer Viskosität von 213 mPa·s bei 25 °C, einem Uretonimingehalt von 8,4 %, einer Farbzahl von 20,6 Jod, einem L*-Wert von 85,0 und einem b*-Wert von 70,3 wurde in einem einstufigen Extraktionsprozess, wie in nachfolgender Vorschrift beschrieben, mit Cyclohexan extrahiert. Polyphenylenpolymethylenpolyisocyanat (BASF: Lupranat^{®} M20) wurde mit Cyclohexan im Verhältnis von Isocyanat : Lösungsmittel von 1 : 3 bei 50 °C für 60 min in Kontakt gebracht. Danach wurde das Produktgemisch bis zur vollständigen Phasenbildung bei Raumtemperatur stehengelassen. Die untere Phase war das sogenannte "Raffinat", das das abzutrennende Uretonimin sowie höherkernige MDI-Homologe enthielt. Die obere Phase war das sogenannte "Extrakt", das das gewünschte uretoniminarme Polyphenylenpolymethylenpolyisocyanat und Lösungsmittel enthielt. Beide Phasen werden getrennt und das Lösungsmittel mittels Vakuumdestillation vollständig entfernt (Restgehalt an Cyclohexan kleiner 20 ppm). Nach der destillativen Entfernung des Extraktionsmittels wurde ein uretoniminarmes Polyphenylenpolymethylenpolyisocyanat (PMDI) mit einem NCO-Gehalt von 33,0 Gew.-%, einer Viskosität von 30 mPa·s bei 25 °C, einer Farbzahl von 0,3 Jod, einem L*-Wert von 99,8 und einem b*-Wert von 1,8 erhalten.

Nach anschließender Entmonomerisierung in einer einstufigen Kurzwegverdampferapparatur wurde ein Monomer-MDI- und uretoniminarmes Polyphenylenpolymethylenpolyisocyanat (PMDI) mit einem NCO-Gehalt von 32,2 Gew.-%, einer Viskosität von 1258 mPa·s bei 25 °C, einem Monomer-MDI-Gehalt von 424 ppm, einem Uretonimingehalt von 2,2 %, einer Farbzahl von 1,6 Jod, einem L*-Wert von 98,1 und einem b*-Wert von 9,7 erhalten.

**Tabelle: Stabilität des uretonimin- und Monomer-MDI-armen Polyphenylenpolymethylenpolyisocyanat verglichen mit einem nur MMDIarmen Polyphenylenpolymethylenpolyisocyanat (Vergleichsbeispiel)**

| | Vergleichsbeispiel | | | PMDI gemäß Ausführungsbeispiel 2 (ai) | | |
|---|---|---|---|---|---|---|
| | Ausgang | nach 2 Wochen bei 40 °C | relative Änderung | Ausgang | nach 2 Wochen bei 40 °C | relative Änderung |
| MMDI-Gehalt | 2669 ppm | 4226 ppm | + 58 % | 1451 ppm | 1873 ppm | + 29 % |
| Viskosität (25 °C) | 30882 mPa·s | 66880 mPa·s | + 117 % | 2865 mPa·s | 3939 mPa·s | + 37 % |
| NCO-Gehalt | 29,9 Gew.-% | 29,5 Gew.-% | - 1 % | 31,6 Gew.-% | 31,5 Gew.-% | - 0,3 % |

### Ausführungsbeispiel 3:

### Fertigung von Isocyanatkomponenten für Einkomponenten- und Zweikomponenten-PUR-Systeme

### 3.1 Isocyanatkomponente für Einkomponenten-PUR-Aerosolschäume

Aus 165 g des monomerfreien MDI-Prepolymers gemäß Ausführungsbeispiel 1, 225 g des uretonimin-armen und Monomer-MDI-armen Polyphenylenpolymethylenpolyisocyanat gemäß Ausführungsbeispiel 2 sowie 110 g Triethylphosphat wurde eine Isocyanatkomponente hergestellt. Diese weist folgende Eigenschaften auf: Isocyanatgehalt: 16,2 Gew.-% NCO; Viskosität bei 25 °C: 150 mPa·s

### 3.2 Isocyanatkomponente für Zweikomponenten-PUR-Hartschäume und PUR-Gießharze

Aus 425 g des uretoniminarmen und Monomer-MDI-armen Polyphenylenpolymethylenpolyisocyanat gemäß Ausführungsbeispiel 2 sowie 75 g Triethylphosphat wurde durch Mischung eine Isocyanatkomponente hergestellt. Diese weist folgende Eigenschaften auf: Isocyanatgehalt: 27,2 Gew.-% NCO; Viskosität bei 25 °C: 98 mPa·s

### Ausführungsbeispiel 4:

### Herstellung eines Einkomponenten-PUR-Aerosolschaumes

### Fertigung der Polyolkomponente

Aus 306 g eines Polyetherpolyols auf Basis Glyzerin/Propylenoxyd/Ethylenoxyd (OH-Zahl: 42 mgKOH/g), 185 g eines Polyetherpolyols auf Basis Glyzerin/Propylenoxyd (OH-Zahl: 155 mgKOH/g), 60 g eines bromierten Polyetherpolyols mit einer OH-Zahl von 330 mgKOH/g (Handelsname IXOL B251 der Firma Solvay Fluor & Derivate, Hannover), 25 g eines Silicon-Schaumstabilisators (Handelsname Tegostab B 2219 der Firma Goldschmidt), 8 g Dimorpholinodiethylether, 330 g Trichlorpropylphosphat, 0,5 g dünnflüssiges Paraffinöl und 0,3 g Siliconöl M100 der Firma Bayer wurde eine Polyolkomponente gefertigt.

### Isocyanatkomponente

Dazu wurde die Isocyanatkomponente gemäß Ausführungsbeispiel 3.1 verwendet.

### Fertigung der Aerosoldose

In eine 1-Liter-Aerosoldose wurden 171 g der Polyolkomponente und nachfolgend 428 g der Isocyanatkomponente eingewogen und die Aerosoldose mit einem Ventil verschlossen.

Durch das Ventil wurden nachfolgend 57 g Dimethylether, 38 g eines 4-bar-Propan/ Butangemisches (90 %Propan und 10 % Butan) sowie 95 g Tetrafluormethan in die Aerosoldose dosiert.

Der Inhalt der Dose wurde durch Schütteln homogenisiert, wobei dadurch die Prepolymerreaktion in Gang gesetzt wurde. Nach 24-h-Lagerung bei 50 °C (alternativ 4 Tage Lagerung bei Raumtemperatur) war die Prepolymerreaktion soweit abgeschlossen, dass der Einkomponenten-PUR-Aerosolschaum verarbeitet werden konnte.

### Herstellung des Schaums

Auf angefeuchtetem Papierflies wurde durch Öffnen des Ventiles der Aerosoldose der Inhalt in Form eines Schaumstranges ausgetragen. Nach ca. 9 min war der Schaum klebfrei, nach ca. 20 min schneidbar und härtete innerhalb von ca. 8 h zu einem Schaum aus, der folgende Eigenschaften aufwies:

| Schaumeigenschaft | | Schaum nach Aus führungsbeispiel 4 | Vergleichsschaum (etwa gleiche Härte) (mit handelsüblichem PMDI her gestellt) |
|---|---|---|---|
| Zugfestigkeit | [N/cm²] | 10 | 8 |
| Dehnung | [%] | 35 | 30 |
| Druckspannung (bei 10% Ver formung) | | 8 | 5 |
| | [N/cm²] | | |
| Schrumpf | [%] | -3,6 | -4 |

### Ausführungsbeispiel 5:

### Herstellung eines Zweikomponenten-PUR-Hartschaumes

### Fertigung der Polyolkomponente

Aus 377 g Lupranol 3424 (Polyetherpolyol auf Basis Saccharose, Pentaerythrit, Diethylenglyol und Propylenoxyd mit einer OH-Zahl von 403 mgKOH/g), 230 g Lupranol 3423 (Polyetherpolyol auf Basis Saccharose, Glycerin und Propylenoxyd mit einer OH-Zahl von 490 mgKOH/g), 20 g Glyzerin, 300 g Lupranol 1100 (Polyetherpolyol auf Basis Propylenglykol und Propylenoxyd mit einer OH-Zahl von 104 mgKOH/g), 54 g Lupranol VP9319 (Polyetherpolyol auf Basis Trimethylolpropan und Propylenoxyd mit einer OH-Zahl von 160 mgKOH/g), 10 g Stabilisator Tegostab B8443, 5 g Stabilisator Niax Silicone SR 393 und 4,5 g Wasser wurde ein Polyolgemisch hergestellt. Diesem Gemisch wurden sowohl 34 g eines Katalysatorgemisches (23,3 % N,N-Dimethylcyclohexylamin, 18,7 % 1-Methylimidazol, 28 % Tetramethylhexandiamin und 30 % Lupranol 1200 [Polyetherpolyol auf Basis Propylenglykol und Propylenoxyd mit einer OH-Zahl von 248 mgKOH/g ]) als auch 50 g eines wässrigen Glyzerin/Glykol-Gemisches (enthaltend 9 % Gyzerin und 31 % Dipropylenglykol) zugesetzt und daraus die Polyolkomponente gefertigt.

### Isocyanatkomponente

Dazu wurde die Isocyanatkomponente gemäß Ausführungsbeispiel 3.2 verwendet.

### Verarbeitung der Komponenten zu PUR-Hartschaum

Die Komponenten wurden im Mischungsverhältnis Polyol- : Isocyanatkomponente = 100 : 164 gemischt und nach Aufschäumen und Aushärtung wurde ein weisser Hartschaum erhalten. Der Schaum hatte dabei (freigeschäumt) folgende Eigenschaften:

| | |
|---|---|
| Startzeit: | 15 sec |
| Abbindezeit: | 46 sec |
| Steigzeit: | 84 sec |
| Raumgewicht: | 43 kg/m³ |
| Druckfestigkeit: | 32 N/cm² |

### Ausführungsbeispiel 6:

### Herstellung eines Zweikomponenten-PUR-Gießharzes

### Fertigung der Polyolkomponente

Aus 730 g eines fettchemischen Polyols auf Basis Sojaöl mit einer Funktionalität von 3,5 und einer Hydroxylzahl von 170 mgKOH/g (Handelsname Sovermol 805), 200 g fettchemischen Polyols auf Basis Sojaöl mit einer Funktionalität von 2,1 und einer Hydroxylzahl von 227 mgKOH/g (Handelsname Sovermol 1102 ), 70 g eines zeolithischen Trockenmittels auf Basis eines Natriumalumosilikats 50 %ig in Rizinusöl sowie 0,5 g eines Silikonentschäumers wurde eine Polyolkomponente hergestellt.

### Isocyanatkomponente 1

Dazu wurde die Isocyanatkomponente gemäß Ausführungsbeispiel 3.2 verwendet.

### Isocyanatkomponente 2 (Vergleichskomponente)

Zur Vergleichszwecken wurde das bisher bekannte handelsübliche Polymer-MDI verwendet.

Die Systemkomponenten wurden mit gleichem Vernetzungsgrad (113 %) umgesetzt.

Vergleich der erzielten mechanischen Eigenschaften:

| Mechanische Eigenschaften | Ausführungsbeispiel 6 (Verwendung der erfindungsgemäßen Isocyanatkomponen te 1) | Vergleichsbeispiel (Verwendung der handelsüblichen Isocyanatkomponente 2) |
|---|---|---|
| Härte [°Shore D] | 55 | 57 |
| Zugfestigkeit [N/mm²] | 22,5 | 24,6 |
| Bruchdehnung [%] | 39 | 31 |
| Weiterreißfestigkeit [N/mm] | 102 | 51 |

## Patentansprüche

1. Verfahren zur Herstellung von Polyurethanen durch Umsetzung von Polyisocyanaten a) mit Verbindungen mit mindestens zwei mit Isocyanatgruppen reaktiven Wasserstoffatomen b), **dadurch gekennzeichnet, dass** als Polyisocyanat a) mindestens ein Gemisch aus Diphenylmethandiisocyanat und Polyphenylenpolymethylenpolyisocyanaten ai) mit einer mittleren Funktionalität von 2,2 bis 5,2, einem Gehalt an 2-Kern MDI von maximal 2 Gew.-%, einem Gehalt an 3-Kern MDI von 25 bis 65 Gew.-%, einem Gehalt an 4-Kern MDI von 5 bis 45 Gew.-%, einem Gehalt an ≥ 5-Kern MDI von 1 bis 40 Gew.-% und einem Gehalt an Uretoniminen von maximal 4 Gew.-%, jeweils bezogen auf das Gewicht des Polyisocyanats ai), eingesetzt wird.

2. Verfahren nach Anspruch 1, **dadurch gekennzeichnet, dass** das Polyisocyanat ai) herstellbar ist, indem Gemische aus Diisocyanaten und Polyisocyanaten mit einer mittleren Funktionalität von größer 2 extrahiert und danach destillativ von Diisocyanaten befreit werden.

3. Verfahren nach Anspruch 1, **dadurch gekennzeichnet, dass** das Polyisocyanat ai) herstellbar ist, indem Gemische aus Diphenylmethandiisocyanat und Polypheriylenpolymethylenpolyisocyanaten mit einer mittleren Funktionalität von größer 2 extrahiert und danach destillativ von Diphenylmethandiisocyanat befreit werden.

4. Verfahren nach Anspruch 1, **dadurch gekennzeichnet, dass** das Polyisocyanat ai) als einziges Polyisocyanat a) eingesetzt wird.

5. Verfahren nach Anspruch 1, **dadurch gekennzeichnet, dass** das Polyisocyanat ai) im Gemisch mit anderen Polyisocyanaten eingesetzt wird.

6. Verfahren nach Anspruch 1, **dadurch gekennzeichnet, dass** das Polyisocyanat ai) im Gemisch mit mindestens einem Isocyanatgruppen und Urethangruppen enthaltenden Prepolymer aii) eingesetzt wird.

7. Verfahren nach Anspruch 6, **dadurch gekennzeichnet, dass** das Prepolymer aii) durch Umsetzung von Polyisocyanaten mit einem stöchiometrischen Unterschuss von Verbindungen mit mindestens zwei mit Isocyanatgruppen reaktiven Wasserstoffatomen hergestellt wird:

8. Verfahren nach Anspruch 6, **dadurch gekennzeichnet, dass** das Prepolymer aii) einen Gehalt an nicht umgesetzten monomeren Isocyanaten von maximal 1 Gew.-%, bezogen auf das Gewicht des Prepolymeren aii), aufweist.

9. Polyisocyanatmischung mit einer mittleren Funktionalität von 2,2 bis 5,2, bestehend aus Diphenylmethandiisocyanat und Polyphenylenpolymethylenpolyisocyanaten mit einem Gehalt an 2-Kern MDI von maximal 2 Gew.-%, einem Gehalt an 3-Kern MDI von 25 bis 65 Gew.-%, einem Gehalt an 4-Kern MDI von 5 bis 45 Gew.-%, einem Gehalt an ≥5-Kern MDI von 1 bis 40 Gew.-% und einem Gehalt an Uretoniminen von maximal 4 Gew.-%, jeweils bezogen auf das Gewicht der Polyisocyanatmischung.

10. Polyisocyanatmischung, enthaltend mindestens ein Polyisocyanat ai) mit einer mittleren Funktionalität von 2,2 bis 5,2, einem Gehalt an 2-Kern MDI von maximal 2 Gew.-%, einem Gehalt an 3-Kern MDI von 25 bis 65 Gew.-%, einem Gehalt an 4-Kern MDI von 5 bis 45 Gew.-%, einem Gehalt an ≥ 5-Kern MDI von 1 bis 40 Gew.-% und einem Gehalt an Uretoniminen von maximal 4 Gew.-%, jeweils bezogen auf das Gewicht des Polyisocyanats ai), und ein Isocyanatgruppen und Urethangruppen enthaltendes Prepolymer aii) mit einem Gehalt an nicht umgesetzten monomeren Isocyanaten von maximal 1 Gew.-%, bezogen auf das Gewicht des Prepolymeren aii).

11. Verwendung der Polyisocyanatmischung nach den Ansprüchen 9 und 10 zur Herstellung von Polyurethanen.

12. Verwendung der Polyisocyanatmischung nach den Ansprüchen 9 und 10 zur Herstellung von 1-Komponenten-Polyurethan-Dosenschäumen.

13. Verwendung der Polyisocyanatmischung nach den Ansprüchen 9 und 10 zur Herstellung von Polyurethan-Kleb- und/oder Dichtstoffen.

14. Verwendung der Polyisocyanatmischung nach den Ansprüchen 9 und 10 zur Herstellung von Polyurethan-Elastomeren.

15. Verwendung der Polyisocyanatmischung nach den Ansprüchen 9 und 10 zur Herstellung von 2-Komponenten-Polyurethanschäumen.

16. Verwendung der Polyisocyanatmischung nach den Ansprüchen 9 und 10 zur Herstellung von 2-Komponenten-Polyurethan-Hartschäumen.

## Claims

1. A process for the preparation of polyurethanes by reacting polyisocyanates a) with compounds having at least two hydrogen atoms reactive with isocyanate groups b), wherein a polyisocyanate a) used is at least one mixture of diphenylmethane diisocyanate and polyphenylenepolymethylene polyisocyanates ai) having an average functionality of from 2.2 to 5.2, a content of dinuclear MDI of not more than 2% by weight, a content of trinuclear MDI of from 25 to 65% by weight, a content of tetranuclear MDI of from 5 to 45% by weight, a content of ≥ pentanuclear MDI of from 1 to 40% by weight, and a content of uretonimines of not more than 4% by weight, based in each case on the weight of the polyisocyanate ai).

2. The process according to claim 1, wherein the polyisocyanate ai) can be prepared by extracting mixtures of diisocyanates and polyisocyanates having an average functionality of greater than 2 and then freeing them from diisocyanates by distillation.

3. The process according to claim 1, wherein the polyisocyanate ai) can be prepared by extracting mixtures of diphenylmethane diisocyanate and polyphenylenepolymethylene polyisocyanates having an average functionality of greater than 2 and then freeing them from diphenylmethane diisocyanate by distillation.

4. The process according to claim 1, wherein the polyisocyanate ai) is used as the only polyisocyanate a).

5. The process according to claim 1, wherein the polyisocyanate ai) is used as a mixture with other polyisocyanates.

6. The process according to claim 1, wherein the polyisocyanate ai) is used as a mixture with at least one prepolymer aii) comprising isocyanate groups and urethane groups.

7. The process according to claim 6, wherein the prepolymer aii) is prepared by reacting polyisocyanates with less than the stoichiometric amount of compounds having at least two hydrogen atoms reactive with isocyanate groups.

8. The process according to claim 6, wherein the prepolymer aii) has a content of unconverted monomeric isocyanates of not more than 1% by weight, based on the weight of the prepolymer aii).

9. A polyisocyanate mixture having an average functionality of from 2.2 to 5.2, consisting of diphenylmethane diisocyanate and polyphenylenepolymethylene polyisocyanates having a content of dinuclear MDI of not more than 2% by weight, a content of trinuclear MDI of from 25 to 65% by weight, a content of tetranuclear MDI of from 5 to 45% by weight, a content of > pentanuclear MDI of from 1 to 40% by weight, and a content of uretonimines of not more than 4% by weight, based in each case on the weight of the polyisocyanate mixture.

10. A polyisocyanate mixture comprising at least one polyisocyanate ai) having an average functionality of from 2.2 to 5.2, a content of dinuclear MDI of not more than 2% by weight, a content of trinuclear MDI of from 25 to 65% by weight, a content of tetranuclear MDI of from 5 to 45% by weight, a content of ≥ pentanuclear MDI of from 1 to 40% by weight, and a content of uretonimines of not more than 4% by weight, based in each case on the weight of the polyisocyanate ai), and a prepolymer aii) comprising isocyanate groups and urethane groups and having a content of unconverted monomeric isocyanates of not more than 1% by weight, based on the weight of the prepolymer aii).

11. The use of the polyisocyanate mixture according to claims 9 and 10 for the preparation of polyurethanes.

12. The use of the polyisocyanate mixture according to claims 9 and 10 for the preparation of 1-component polyurethane spray foams.

13. The use of the polyisocyanate mixture according to claims 9 and 10 for the preparation of polyurethane adhesives and/or sealing compounds.

14. The use of the polyisocyanate mixture according to claims 9 and 10 for the preparation of polyurethane elastomers.

15. The use of the polyisocyanate mixture according to claims 9 and 10 for the preparation of 2-component polyurethane foams.

16. The use of the polyisocyanate mixture according to claims 9 and 10 for the preparation of 2-component rigid polyurethane foams.

## Revendications

1. Procédé pour la préparation de polyuréthanes par transformation de polyisocyanates a) avec des composés présentant au moins deux atomes d'hydrogène b) réactifs avec des groupes isocyanate, **caractérisé en ce qu'**on utilise comme polyisocyanate a) au moins un mélange de diphénylméthanediisocyanate et de polyphénylène-polyméthylène-polyisocyanates ai) avec une fonctionnalité moyenne de 2,2 à 5,2, une teneur en MDI à 2 noyaux d'au maximum 2% en poids, une teneur en MDI à 3 noyaux de 25 à 65% en poids, une teneur en MDI à 4 noyaux de 5 à 45% en poids, une teneur en MDI à ≥ 5 noyaux de 1 à 40% en poids et une teneur en urétonimines d'au maximum 4% en poids, à chaque fois par rapport au poids du polyisocyanate ai).

2. Procédé selon la revendication 1, **caractérisé en ce que** le polyisocyanate ai) peut être préparé **en ce que** des mélanges de diisocyanates et de polyisocyanates présentant une fonctionnalité moyenne supérieure à 2 sont extraits puis libérés par distillation de diisocyanates.

3. Procédé selon la revendication 1, **caractérisé en ce que** le polyisocyanate ai) peut être préparé **en ce que** des mélanges de diphénylméthanediisocyanate et de polyphénylène-polyméthylène-polyisocyanates présentant une fonctionnalité moyenne supérieure à 2 sont extraits puis libérés par distillation de diphénylméthanediisocyanates.

4. Procédé selon la revendication 1, **caractérisé en ce que** le polyisocyanate ai) est utilisé comme polyisocyanate a) unique.

5. Procédé selon la revendication 1, **caractérisé en ce que** le polyisocyanate ai) est utilisé en mélange avec d'autres polyisocyanates.

6. Procédé selon la revendication 1, **caractérisé en ce que** le polyisocyanate ai) est utilisé en mélange avec au moins un prépolymère aii) contenant des groupes isocyanate et uréthane.

7. Procédé selon la revendication 6, **caractérisé en ce que** le prépolymère aii) est préparé par transformation de polyisocyanates avec une quantité inférieure à la quantité stoechiométique de composés présentant au moins deux atomes d'hydrogène réactifs avec des groupes isocyanate.

8. Procédé selon la revendication 6, **caractérisé en ce que** le prépolymère aii) présente une teneur en isocyanates monomères non transformés d'au maximum 1% en poids par rapport au poids du prépolymère aii).

9. Mélange de polyisocyanates présentant une fonctionnalité moyenne de 2,2 à 5,2, constitué de diphénylméthanediisocyanate et de polyphénylène-polyméthylène-polyisocyanates présentant une teneur en MDI à 2 noyaux d'au maximum 2% en poids, une teneur en MDI à 3 noyaux de 25 à 65% en poids, une teneur en MDI à 4 noyaux de 5 à 45% en poids, une teneur en MDI à ≥ 5 noyaux de 1 à 40% en poids et une teneur en urétonimines d'au maximum 4% en poids, à chaque fois par rapport au poids du mélange de polyisocyanates.

10. Mélange de polyisocyanates, contenant au moins un polyisocyanate ai) présentant une fonctionnalité moyenne de 2,2 à 5,2, une teneur en MDI à 2 noyaux d'au maximum 2% en poids, une teneur en MDI à 3 noyaux de 25 à 65% en poids, une teneur en MDI à 4 noyaux de 5 à 45% en poids, une teneur en MDI à ≥ 5 noyaux de 1 à 40% en poids et une teneur en urétonimines d'au maximum 4% en poids, à chaque fois par rapport au poids du polyisocyanate ai), et un prépolymère aii) contenant des groupes isocyanate et uréthane présentant une teneur en isocyanates monomères non transformés d'au maximum 1% en poids, par rapport au poids du prépolymère aii).

11. Utilisation du mélange de polyisocyanates selon les revendications 9 et 10 pour la préparation de polyuréthanes.

12. Utilisation du mélange de polyisocyanates selon les revendications 9 et 10 pour la préparation de mousse en bombe de polyuréthane à 1 composant.

13. Utilisation du mélange de polyisocyanates selon les revendications 9 et 10 pour la préparation d'adhésifs et/ou de substances d'étanchéité en polyuréthane.

14. Utilisation du mélange de polyisocyanates selon les revendications 9 et 10 pour la préparation d'élastomères de polyuréthane.

15. Utilisation du mélange de polyisocyanates selon les revendications 9 et 10 pour la préparation de mousses de polyuréthane à 2 composants.

16. Utilisation du mélange de polyisocyanates selon les revendications 9 et 10 pour la préparation de mousses dures de polyuréthane à 2 composants.
